(19) Europäisches Patentamt
European Patent Office
Office européen des brevets

(11) **EP 4 643 992 A1**

(12) **EUROPEAN PATENT APPLICATION**
published in accordance with Art. 153(4) EPC

(43) Date of publication:
**05.11.2025 Bulletin 2025/45**

(21) Application number: **22970143.8**

(22) Date of filing: **28.12.2022**

(51) International Patent Classification (IPC):
***B01J 37/00*** (2006.01)

(52) Cooperative Patent Classification (CPC):
**B01J 37/00**

(86) International application number:
**PCT/JP2022/048515**

(87) International publication number:
**WO 2024/142355 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Kao Corporation
Chuo-ku,
Tokyo 103-8210 (JP)**

(72) Inventors:
• **SAKAI, Kento
Wakayama-shi, Wakayama 640-8404 (JP)**
• **TAKEDA, Kosuke
Wakayama-shi, Wakayama 640-8404 (JP)**
• **SEKI, Tomotaka
Wakayama-shi, Wakayama 640-8404 (JP)**
• **FURIKADO, Ippei
Wakayama-shi, Wakayama 640-8404 (JP)**

(74) Representative: **Hoffmann Eitle
Patent- und Rechtsanwälte PartmbB
Arabellastraße 30
81925 München (DE)**

(54) **SCREENING METHOD**

(57) [Object] To efficiently screen alloys useful as solid catalysts.

[Solving Means] In an embodiment of the present invention, screening is performed for an alloy that causes a target catalytic reaction. For each of a plurality of candidate elements, basic information including respective states of each reaction substrate in a non-adsorbed state and an energy and a vibration frequency of each reaction substrate in an adsorbed state and each atom constituting the reaction substrate in a plurality of elementary reactions is created, and a plurality of activation energies, a plurality of differential energies, and a reaction rate are obtained. Linearity is evaluated for each of the activation energies and each of the plurality of differential energies, and a feature amount energy from which high linearity is obtained for all of the plurality of activation energies is selected from the plurality of differential energies. Distribution information indicating distribution of the reaction rate with respect to the feature amount energy is created. A plurality of compositions that increases the reaction rate when forming the alloy is predicted. A stability index and an activity index are calculated for each of a plurality of slab models created for the plurality of compositions.

FIG.2

EP 4 643 992 A1

## Description

Technical Field

The present invention relates to a method of screening alloys useful as solid catalysts.

Background Art

Improving the performance of solid catalysts is becoming important not only for the development of the chemical industry but also for the realization of a sustainable society. In order to improve the performance of solid catalysts, for example, many attempts have been made to form an alloy by adding a different element to a solid catalyst formed of a single element. However, in the case of the solid catalyst formed of an alloy, the performance changes greatly depending on structural elements such as the constituent elements and atomic arrangement, and the number of combinations of these structural elements can be said to be substantially infinite. For this reason, it is not realistic to find a solid catalyst capable of providing high performance only through experimental trial and error.

In this regard, attempts have been made to efficiently search for high performance solid catalysts by utilizing computational chemistry such as quantum chemical calculations. For example, Patent Literature 1 discloses a technology for finding a solid catalyst with high the activity of the catalytic reaction that promotes the reaction of a second gas in the present of a first gas. In this technology, the base material element and doping element constituting the solid catalyst are identified on the basis of the amount of change in the adsorption energy of the first gas and the second gas depending on the presence or absence of the doping element.

Citation List

Patent Literature

Patent Literature 1: Japanese Patent Application Laid-open No. 2020-006312

Disclosure of Invention

Technical Problem

However, in the technology described in Patent Literature 1, only the adsorption energy when the base material element and a limited doping element are combined is calculated, and the reaction rate of the catalytic reaction is not directly taken into consideration. Further, the technology described in Patent Literature 1 does not provide an indicator for which element to select and is not efficient when there are many candidate elements and when considering variations in combination. Further, there is a demand for a technology for screening solid catalysts that is applicable not only to a specific catalytic reaction including a single elementary reaction as in the technology described in Patent Literature 1 but also to a catalytic reaction including a plurality of elementary reactions such as a rection that promotes the reaction in a production process using a plurality of raw materials and a catalytic reaction where other factors need to be taken into consideration.

An object of the present invention is to efficiently screen alloys useful as solid catalysts. Solution to Problem

A screening method according to an embodiment of the present invention performs screening for an alloy that causes a target catalytic reaction.

For each of a plurality of candidate elements that is a candidate for a metal element constituting the alloy, basic information including respective states of each reaction substrate in a non-adsorbed state and an energy and a vibration frequency of each reaction substrate in an adsorbed state and each atom constituting the reaction substrate in a plurality of elementary reactions constituting the catalytic reaction, for a specific crystal plane formed by each of the candidate elements, is created.

For each of the plurality of candidate elements, a plurality of activation energies corresponding to the plurality of elementary reactions, a plurality of differential energies obtained as relative values of energies in the respective states, and a reaction rate of the catalytic reaction from the basic information are obtained.

For each activation energy constituting the plurality of activation energies and each differential energy constituting the plurality of differential energies, linearity is evaluated from a relationship between the plurality of candidate elements and a feature amount energy from which high linearity is obtained for all of the plurality of activation energies is selected from the plurality of differential energies.

Distribution information indicating distribution of the reaction rate with respect to the feature amount energy is created using the feature amount energy and the reaction rate in the plurality of candidate elements.

By using the distribution information, a plurality of compositions that contains at least two candidate elements of the plurality of candidate elements and increases the reaction rate when forming the alloy is predicted.

A plurality of slab models corresponding to the plurality of compositions is created.

A stability index is calculated on a basis of an energy calculated for each of the plurality of slab models.

An activity index is calculated on a basis of the reaction rate obtained from the distribution information using the feature amount energy calculated for each of the plurality of slab models.

An information processing apparatus according to an embodiment of the present invention comprises: a processing unit that executes the following.

For each of a plurality of candidate elements that is a candidate for a metal element constituting the alloy, basic information including respective states of each reaction substrate in a non-adsorbed state and an energy and a vibration frequency of each reaction substrate in an adsorbed state and each atom constituting the reaction substrate in a plurality of elementary reactions constituting the catalytic reaction, for a specific crystal plane formed by each of the candidate elements, is created.

For each of the plurality of candidate elements, a plurality of activation energies corresponding to the plurality of elementary reactions, a plurality of differential energies obtained as relative values of energies in the respective states, and a reaction rate of the catalytic reaction from the basic information are obtained.

For each activation energy constituting the plurality of activation energies and each differential energy constituting the plurality of differential energies, linearity is evaluated from a relationship between the plurality of candidate elements and a feature amount energy from which high linearity is obtained for all of the plurality of activation energies is selected from the plurality of differential energies.

Distribution information indicating distribution of the reaction rate with respect to the feature amount energy is created using the feature amount energy and the reaction rate in the plurality of candidate elements.

By using the distribution information, a plurality of compositions that contains at least two candidate elements of the plurality of candidate elements and increases the reaction rate when forming the alloy is predicted.

A plurality of slab models corresponding to the plurality of compositions is created.

A stability index is calculated on a basis of an energy calculated for each of the plurality of slab models.

An activity index is calculated on a basis of the reaction rate obtained from the distribution information using the feature amount energy calculated for each of the plurality of slab models.

A program according to an embodiment of the present invention causes an information processing to execute the following.

For each of a plurality of candidate elements that is a candidate for a metal element constituting the alloy, basic information including respective states of each reaction substrate in a non-adsorbed state and an energy and a vibration frequency of each reaction substrate in an adsorbed state and each atom constituting the reaction substrate in a plurality of elementary reactions constituting the catalytic reaction, for a specific crystal plane formed by each of the candidate elements, is created.

For each of the plurality of candidate elements, a plurality of activation energies corresponding to the plurality of elementary reactions, a plurality of differential energies obtained as relative values of energies in the respective states, and a reaction rate of the catalytic reaction from the basic information are obtained.

For each activation energy constituting the plurality of activation energies and each differential energy constituting the plurality of differential energies, linearity is evaluated from a relationship between the plurality of candidate elements and a feature amount energy from which high linearity is obtained for all of the plurality of activation energies is selected from the plurality of differential energies.

Distribution information indicating distribution of the reaction rate with respect to the feature amount energy is created using the feature amount energy and the reaction rate in the plurality of candidate elements.

By using the distribution information, a plurality of compositions that contains at least two candidate elements of the plurality of candidate elements and increases the reaction rate when forming the alloy is predicted.

A plurality of slab models corresponding to the plurality of compositions is created.

A stability index is calculated on a basis of an energy calculated for each of the plurality of slab models.

An activity index is calculated on a basis of the reaction rate obtained from the distribution information using the feature amount energy calculated for each of the plurality of slab models.

Advantageous Effects of Invention

According to the present invention, it is possible to efficiently screen alloys useful as solid catalysts.

Brief Description of Drawings

[Fig. 1] Fig. 1 is a flowchart showing a screening method according to an embodiment of the present invention.
[Fig. 2] Fig. 2 is a diagram illustrating distribution information created in Step S05 of the screening method.
[Fig. 3] Fig. 3 is a diagram describing Step S06 of the screening method.
[Fig. 4] Fig. 4 is a diagram describing Step S06 of the screening method.
[Fig. 5] Fig. 5 is a diagram illustrating a list of alloys that can be used in Step S10 of the screening method.
[Fig. 6] Fig. 6 is a block diagram showing an information processing apparatus capable of realizing the screening method.

Mode(s) for Carrying Out the Invention

[Introduction]

A screening method according to an embodiment of the present invention is configured to be capable of efficiently searching for alloys useful as solid catalysts that cause a target catalytic reaction by calculation using an information processing apparatus. By feeding back the search results by the screening method according to this embodiment, it is possible to narrow down the number of alloys to be evaluated by experiments among alloys with the huge number of combinations of structural elements. This makes it possible to design high performance solid catalysts with less effort and cost, and a solid catalyst having excellent performance and is more

The screening method according to this embodiment uses, as an index for searching for alloys useful as solid catalysts, not only the activity of the catalytic reaction of the alloy but also the stability of the structure of the alloy. This makes it possible to select an alloy that can provide high performance as a solid catalyst from among compositions that are capable of realizing stable structures. Therefore, in the screening method according to this embodiment, it is possible to exclude, from the experimental evaluation, configurations that are difficult to prepare or are prone to structural changes over time. This makes it possible to efficiently search for alloys useful as solid catalysts that cause a target catalytic reaction.

[Configuration of entire screening method]

(Overview)

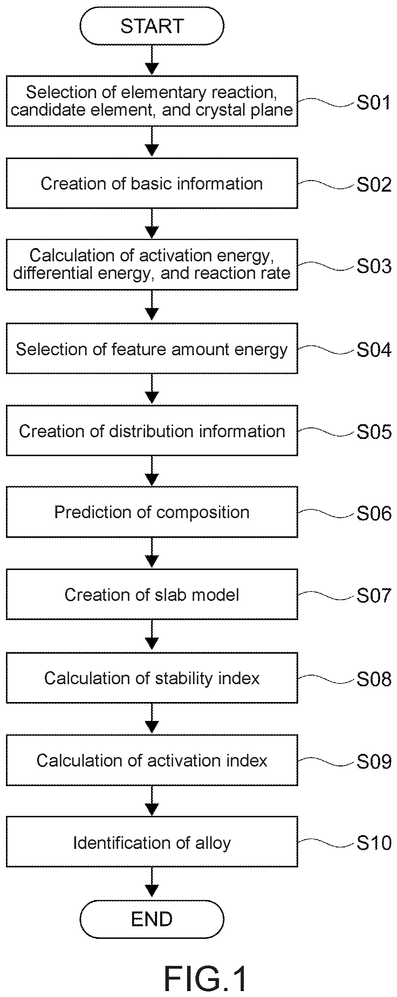

The configuration of the entire screening method according to this embodiment will be described below. The screening method according to this embodiment includes Step S01 to Step S10 shown in Fig. 1. Step S01 to Step S10 of the screening method according to this embodiment will be described below in detail with reference to Fig. 1.

(Step S01: Selection of elementary reaction, candidate element, and crystal plane)

In Step S01, first, a target catalytic reaction is selected. The catalytic reaction refers to a series of chemical reactions that proceed or are promoted by the action of a catalyst. The target catalytic reaction can be realized by a solid catalyst and can be arbitrarily selected from those including successive reactions on the same catalyst. Further, even in the case where the substrate is desorbed during the reaction, the target catalytic reaction can be selected separately before and after the desorption. Further, the target catalytic reaction may be either a liquid phase reaction or a gas phase reaction. In addition, as the energy to be applied for the target catalytic reaction, a thermal energy or a mechanical energy is suitable. Other examples include an electrical energy and a light energy. Examples of the method of applying the thermal energy include microwave heating, in addition to heater heating. Examples of the method applying the mechanical energy include application of pressure. Further, examples of the target catalytic reaction include an exchange reaction in which some molecule or groups of a compound are exchanged, an addition reaction in which some molecule or groups of a compound are added, and a decomposition reaction. Examples of the exchange reaction and the addition reaction include an oxidation reaction, a hydrogenation reaction, an isomerization reaction, and a cyclization reaction. Examples of the decomposition reaction include a desulfurization reaction, a deoxygenation reaction, and a dehydrogenation reaction. Specific examples of the target catalytic reaction selected in Step S01 include an oxidation reaction of carbon monoxide, a transesterification reaction between an ester and an alcohol, an esterification reaction between a fatty acid and an alcohol, a reaction for producing an alcohol by hydrogenation of an ester, a reaction for producing a substituted amine by an amine and an alcohol, a hydrogenation reaction of a fatty acid, a hydrogenolysis reaction of an alcohol, a hydrodesulfurization reaction of sulfur, a cross-aldol reaction of an aldehyde, a hydrogenation reaction of an aromatic compound, a selective hydrogenation reaction of olefin, a hydrogenation reaction of a carbonyl compound, a hydroisomerization reaction of olefin, an ene cyclization reaction of diene, an oxygen-oxidation reaction of an alcohol, and a hydrogenation reaction of carbon monoxide or carbon dioxide (including a hydrogenation reaction accompanied by a carbon chain elongation reaction).

Next, a plurality of elementary reactions constituting the selected catalytic reaction is selected. The elementary reaction is each reaction in each stage when the catalytic reaction is divided into a plurality of stages. That is, the plurality of elementary reactions can be selected in accordance with the target catalytic reaction and can be estimated from, for example, description in literature. Further, the plurality of elementary reactions can include possible reactions as candidates in addition to the estimated reaction.

Further, a plurality of candidate elements that is a candidate for a metal element that causes the selected catalytic reaction is selected. As the candidate element, any two or more metal elements can be selected from all metal elements. For example, a set of metal elements commonly used as solid catalysts can be selected. From the viewpoint of screening accuracy, the number of candidate elements is favorably three or more. Further, the number of candidate elements is favorably 30 or less from the viewpoint of screening efficiency.

In addition, a crystal structure that causes the selected catalytic reaction is selected. The crystal structure can be arbitrarily selected from crystal structures forming a body-centered cubic lattice, a face-centered cubic lattice, and a hexagonal close-packed structure. For example, a general crystal structure that causes the catalytic reaction can be selected. Further, for the crystal structure, any crystal plane represented by Miller indices, e.g., the general (111) plane, can be selected, and two or more crystal planes can also be selected. Further, as the crystal plane, for example, a crystal plane that is expected to exist on the nanoparticles in equilibrium by calculating the surface formation free energy by quantum chemical calculations and using the Wulff plot or referring to a database such as Crystalium can be selected.

As an example, the case where an oxidation reaction of CO ($CO + 0.5O_2 \rightarrow CO_2$) is selected as the target catalytic reaction will be described. Assumption is made that this catalytic reaction includes three elementary reactions with the respective reaction substrates shown in the following formulae. Here, the "reaction substrate" refers to a compound whose reaction proceeds or is promoted by the action of a catalyst, which is determined in accordance with the type of catalytic reaction. Note that "*" in each formula represents the adsorption site on the solid catalyst surface.

$$* + CO \rightarrow CO^*$$

$$2^* + O_2 \Leftrightarrow O\text{-}O^* + ^* \rightarrow 2O^*$$

$$CO^* + O^* \Leftrightarrow O\text{-}CO^* + ^* \rightarrow CO_2 + 2^*$$

Note that "$\Leftrightarrow$" in the above formulae represents a "symbol combining an arrow pointing to the right $\rightarrow$ and an arrow pointing to the left $\leftarrow$".

Further, the plurality of candidate elements that is the candidate for a metal element that causes this catalytic reaction can be selected from Ru, Ni, Rh, Cu, Pd, Pt, Ag, Au, and the like, which are general elements constituting solid catalysts. Further, as a crystal plane that causes this catalytic reaction, the (111) plane, which is common as a crystal plane that causes the catalytic reaction, or the like can be selected, and two or more crystal planes can also be selected.

Note that in the screening method according to this embodiment, at least part of Step S01 can be automatically performed by an information processing apparatus. That is, in Step S01, when a target catalytic reaction is input, at least one of a plurality of elementary reactions, a plurality of candidate elements, a plurality of adsorption structures, or a crystal plane may be automatically identified from a database.

Further, in Step S01, it does not necessarily need to identify at least one of a plurality of elementary reactions, a plurality of candidate elements, a plurality of adsorption structures, and a crystal plane each time, and data prepared in advance may be used. For example, in the information processing apparatus, the past data identified for the catalytic reaction may be stored in a storage unit, and in the case where past data regarding the same catalytic reaction exists in the storage unit, the data may be read from the storage unit.

(Step S02: Creation of basic information)

In Step S02, basic information is created. The basic information is information that is the basis for calculating the activation energy, the differential energy, and the reaction rate in Step S03.

The basic information created in Step S02 includes a state of each reaction substrate in a non-adsorbed state and an energy and a vibration frequency of each reaction substrate in an adsorbed state and each atom constituting the reaction substrate, in a plurality of elementary reactions calculated for each candidate element. Each state in the plurality of elementary reactions indicates an initial state, a final state, and a transition state of each reaction substrate in each elementary reaction. The adsorbed state of each atom constituting the reaction substrate indicates the adsorbed state in the case where each of the atoms constituting the reaction substrate is present alone. Each state in the plurality of elementary reactions and the energy and vibration frequency of each atom constituting the reaction substrate can be calculated using a general quantum chemical calculation method or a calculation method with accuracy equivalent to that of quantum chemical calculations. Examples of the quantum chemical calculation method that can be used to calculate the energy and vibration frequency of each reaction substrate in each state in the plurality of elementary reactions include an electronic state calculation method based on density functional theory (DFT). Examples of the method of calculating the transition state include a nudged elastic band (NEB) method. Further, examples of the calculation method with accuracy equivalent to that of quantum chemical calculations include a molecular dynamics calculation method based on a machine learning potential.

Further, the basic information created in Step S02 includes conditions of the catalytic reaction. Examples of the conditions of the catalytic reaction include a temperature and a gas partial pressure. The conditions of the catalytic reaction can be determined on the basis of the environment in which the catalytic reaction is actually caused. By determining the conditions of the catalytic reaction on the basis of the actual environment in this way, it is possible to find an alloy that is more suitable for the intended use of the solid catalyst. In the catalytic reaction assumed in this application, it is favorable to identify both the temperature and the gas partial pressure as basic information for the conditions of the catalytic reaction.

As an example, in the case where the target catalytic reaction is the oxidation reaction of CO similarly to the above, the adsorbed states of the respective reaction substrates of the plurality of elementary reactions specified in Step S01 and the atom the constituting the reaction substrate include the following 10 states.

CO (gas alone)
$CO_2$ (gas alone)
$O_2$ (gas alone)
$CO_2^*$ (adsorbed state)
$CO^*$ (adsorbed state)
$O_2^*$ (adsorbed state)
$O^*$ (adsorbed state)
$C^*$ (adsorbed state)
$O\text{-}O^*$ (transition state)
$O\text{-}CO^*$ (transition state)

The energy and vibration frequency for each of the 10 states are calculated using the quantum chemical calculation method. Further, the conditions of the catalytic reaction can be calculated on the basis of the environment that actually promotes the oxidation of CO, such as a place where CO is supplied.

(Step S03: Calculation of activation energy, differential energy, and reaction rate)

In Step S03, the activation energy, the differential energy, and the reaction rate are calculated for each candidate element from the basic information created in Step S02. The activation energy, the differential energy, and the reaction rate are required to create distribution information in Step S05.

The reaction rate is calculated for each candidate element and is a physical quantity indicating the activity of the catalytic reaction of each candidate element. The reaction rate of the catalytic reaction of each candidate element can be calculated using the energy and vibration frequency in each state in the plurality of elementary reactions for each candidate element and the conditions of the temperature and the gas partial pressure of the catalytic reaction, which are included in the basic information created in Step S02. The reaction rate of the catalytic reaction of each candidate element can be calculated using a general algorithm. Examples of the algorithm capable of calculating the reaction rate include a Newton method.

The plurality of activation energies is calculated corresponding to the plurality of elementary reactions for each candidate element. Each of the activation energies is a physical quantity indicating the energy required to cause each elementary reaction in each candidate element. The activation energy of each elementary reaction can be calculated using the energy in each state in the plurality of elementary reactions for each candidate element, which is included in the basic information created in Step S02.

The differential energy is obtained as a relative value of the energy of two states included in the plurality of elementary reactions for each candidate element. The differential energy is a physical quantity that is a candidate of the feature amount energy to be determined in Step S04. The differential energy is calculated as a difference between energies in any two states included in the plurality of elementary reactions. As the differential energy, for example, an adsorption energy or an energy of an intermediate can be used. Note that the adsorption energy is a differential energy calculated as a difference between energies when being adsorbed to each candidate element and when being in a gas state, of the reaction substrate and each atom constituting the reaction substrate.

(Step S04: Selection of feature amount energy)

In Step S04, from the plurality of differential energies calculated in Step S03, a feature amount energy to be used as feature amount for promoting the reaction by being associated with the reaction rate is selected. Specifically, in Step S04, linearity is evaluated from the relationship between the plurality of candidate elements for all combinations of the plurality of activation energies and the plurality of differential energies. Then, from the plurality of differential energies, a differential energies from which high linearity is obtained for all of the plurality of activation energies is selected as a feature amount energy.

Here, the feature amount energy from which high linearity is obtained for all of the plurality of activation energies also has high linearity with respect to the logarithm of the reaction rate. This is based on the fact that the activation energies of all elementary reactions constituting the catalytic reaction have a linear relationship relative to the logarithm of the reaction rate of each elementary reaction in accordance with the Arrhenius equation, and therefore also have a linear relationship relative to the entire reaction rate as a combination of the elementary reactions. For this reason, by associating the feature amount energy selected in Step S04 with the reaction rate, it is possible to convert the feature amount energy into a reaction rate.

Specifically, the linearity of an activation energy $E_{a1}$ included in the plurality of activation energies and a differential energy $E_{b1}$ included in the plurality of differential energies can be evaluated from the linearity of the plots in the scatter plot obtained by plotting the activation energy $E_{a1}$, and the differential energy $E_{b1}$ of all candidate elements on a two-dimensional graph with the activation energy $E_{a1}$ and each differential energy $E_{b1}$ as the two axes.

The linearity of the plots for selecting the feature amount energy from which high linearity is obtained can be evaluated using, for example, a coefficient of determination $r^2$ in the least squares method as an index. In the evaluation using the coefficient of determination $r^2$ as an index, for example, by listing the plurality of differential energies in descending order of the coefficient of determination $r^2$ (or descending order of the numerical value) and dividing the plurality of differential energies into groups of high and low numerical values of the coefficient of determination $r^2$, a differential energy that belongs to the high group can be selected as the feature amount energy. At this time, when grouping the plurality of differential energies, for example, differential energies with the coefficient of determination $r^2$ of a predetermined threshold value or more, favorably 0.7 or more, more favorably 0.8 or more, can be grouped as a high group. Further, for the plurality of differential energies listed in descending order of the coefficient of determination $r^2$, differential energies with a predetermined rank or more can also be selected as feature amount energies. Note that the method of selecting the feature amount energy from the plurality of differential energies is not limited to the above, and any known method can be used.

The number of feature amount energies different from each other selected from the plurality of differential energies in Step S04 can be arbitrarily calculated in accordance with the number of dimensions of the distribution information created in Step S05. That is, in the case where N-dimensional distribution information is created in Step S05, N feature amount energies different from each other are selected in Step S04.

As an example, in the case where the target catalytic reaction is the oxidation reaction of CO similarly to the above and two-dimensional distribution information that is easy to understand visually is created in Step S05, for example, the adsorption energy of CO and the adsorption energy of O can be selected as two feature amount energies different from each other from the plurality of differential energies.

(Step S05: Creation of distribution information)

In Step S05, distribution information that is a graph indicating the distribution of reaction rates with respect to the feature amount energy selected in Step S04 is created. The distribution information is information that shows the feature amount energy and the reaction rate in a continuous manner, i.e., associates any feature amount energy with a reaction rate. In Step S05, in addition to the plots of the plurality of candidate elements obtained from the reaction rate calculated in Step S03 and the feature amount energy selected in Step S04, regions where no plots exist can be interpolated by calculating the reaction rate from the feature amount energy at any resolution to create distribution information.

That is, according to the distribution information, it is possible to obtain a reaction rate from the feature amount energy that can be easily calculated as a relative value of an energy without using complex calculations. With the distribution information, it is possible to obtain the reaction rate from the feature amount energy for any metal and obtain the reaction rate from the feature amount energy for not only an elemental metal but also an alloy having any composition. Therefore, according to the distribution information, it is possible to easily obtain the reaction rate of any alloy.

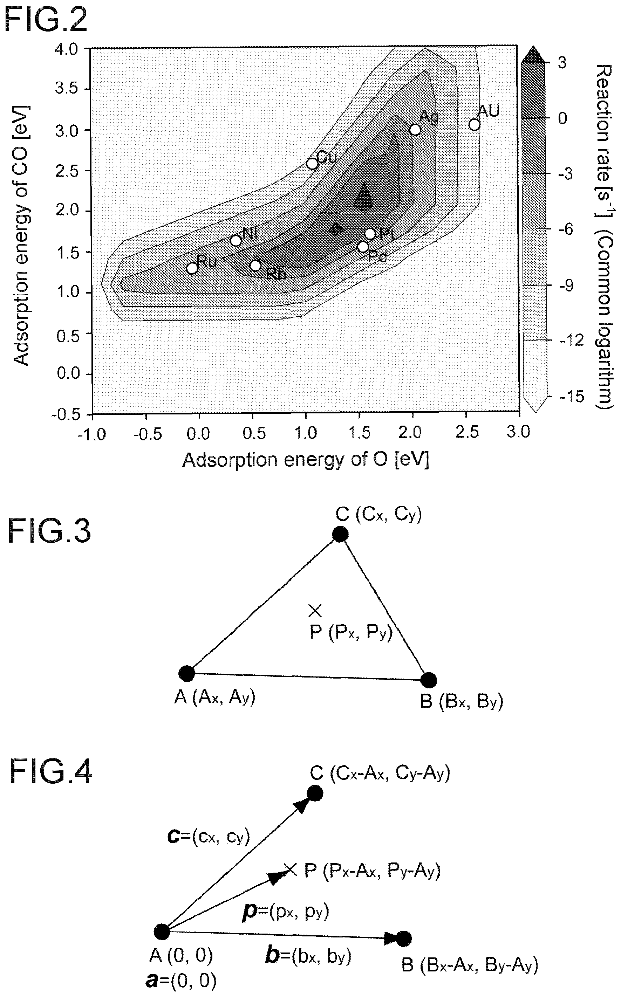

As an example, Fig. 2 shows two-dimensional distribution information in the case where the target catalytic reaction is the oxidation reaction of CO similarly to the above and the feature amount energy is the adsorption energy of CO and the adsorption energy of O. The distribution information shown in Fig. 2 is shown as a two-dimensional heatmap in which the reaction rate (common logarithm) is displayed as different colors in a graph with the adsorption energy of CO and the adsorption energy of O as the vertical axis and the horizontal axis, respectively. Creating the distribution information as a heatmap in this way makes regions where a high reaction rate is obtained easier to be visually recognized.

(Step S06: Prediction of composition)

In Step S06, the composition of a plurality of alloys that contains at least two of the plurality of candidate elements and is likely to obtain a higher reaction rate is obtained (combinations of candidate elements constituting the alloy and the composition ratio of each candidate element in the combination) is predicted on the basis of the distribution information created in Step S05. Specifically, in Step S06, the composition of an alloy that will provide a high reaction rate is predicted from the positional relationship between a target plot P corresponding to the target value of the reaction rate in the distribution information and the plot of each candidate element. The target plot P is usually set at the position where the reaction rate is the highest. For example, in the heatmap shown in Fig. 2, the target plot P can be set in the darkest region. The composition of an alloy that will provide a reaction rate close to the target plot P can be predicted using, for example, the plots of the plurality of candidate elements in the distribution information and coordinate information of the target plot P. Using the coordinate information in this way allows the composition of an alloy that will provide a high reaction rate to be predicted more mechanically and with higher accuracy.

As an example, the case where the composition of an alloy is a ternary system including candidate elements A, B, and C will be described. In this case, for example, two-dimensional distribution information created from two feature amount energies can be used.

Fig. 3 shows plots $A (A_x, A_y), B (B_x, B_y), C (C_x, C_y)$ of the candidate elements A, B, and C and the target plot P ($P_x$, $P_y$) displayed on the distribution information. That is, the two feature amount energies of the candidate element A are $A_x$ and $A_y$, the two feature amount energies of the candidate element B are $B_x$ and $B_y$, and the two feature amount energies of the candidate element C are $C_x$ and $C_y$. Further, the two feature amount energies of the target plot P are $P_x$ and $P_y$.

Here, as shown in Fig. 4, when the plot A is the origin, the plot B can be represented by a vector b (($b_x, b_y$) = ($B_x - A_x$, $B_y - A_y$)), the plot C can be represented by a vector c (($c_x, c_y$) = ($C_x - A_x$, $C_y - A_y$)), and the target plot P can be represented by a vector p (($p_x, p_y$) = ($P_x - A_x$, $P_y - A_y$)). Note that the plot A can be represented by a zero vector a (0, 0). At this time, the vector p can be represented by the following formula using the vectors b and c and coefficients $\beta$ and $\gamma$.

$$p = \beta b + \gamma c$$

This formula can be converted into the following formula using a coefficient matrix D.

(Math. 1)

$$\begin{pmatrix} p_x \\ p_y \end{pmatrix} = D \begin{pmatrix} \beta \\ \gamma \end{pmatrix}$$

$$\begin{pmatrix} \beta \\ \gamma \end{pmatrix} = D^{-1} \begin{pmatrix} p_x \\ p_y \end{pmatrix}$$

Wherein, $$D = \begin{pmatrix} b_x & c_x \\ b_y & c_y \end{pmatrix}$$

In this case, in the case where all the conditions of $\beta + \gamma < 1$, $\beta > 0$, and $\gamma > 0$ are satisfied, the composition ratio A:B:C of the three candidate elements A, B, and C can be calculated as "1-($\beta+\gamma$):$\beta$:$\gamma$". Note that satisfying the above conditions means that the target plot P is located inside the triangle with the vertices of the plots A, B, and C shown in Fig. 3. In this embodiment, the composition in which the target plot P is present within the region surrounded by the lines connecting the plots of the candidate elements can be set as a candidate for the composition of an alloy that is likely to have a high reaction rate. That is, compositions in which the target plot P is not present within the region surrounded by the lines connecting the plots of the candidate elements can excluded from the candidates for the composition of an alloy that is likely to have a high reaction rate.

Further, an index m is calculated from the coefficients $\beta$ and $\gamma$ obtained above on the basis of the following formula.

(Math. 2)

$$m = \left(\beta - \frac{1}{3}\right)^2 + \left(\gamma - \frac{1}{3}\right)^2$$

The index m represents the square of the distance between the center of gravity of the triangle with the vertices of the plots A, B, and C and the target plot P. For this reason, as the index m of the composition becomes smaller, the ratio of each candidate element is closer, making it possible to predict the composition of an alloy that will provide a high reaction rate, which is difficult to predict from the feature amount energy of a single element. Therefore, as a prediction result of the composition of an alloy having a close ratio of each candidate element, a predetermined number of compositions can be listed in ascending order of the index m. In this way, in this embodiment, it is possible to list, for an alloy containing a plurality of candidate elements, a predetermined number of compositions in ascending order of the distance between the estimated plot on the distribution information and the target plot P.

Next, the case where the composition of an alloy is an n-element system including a combination of four or more candidate elements $A_1$, $A_2$, ..., $A_{(n-1)}$, and $A_n$ will be described. In this case, for example, $(n-1)$-dimensional distribution information created from $(n-1)$ feature amount energies can be used.

Plots $A_1$ ($A_{11}$, $A_{12}$, ..., $A_{1(n-2)}$, and $A_{1(n-1)}$), $A_2$ ($A_{21}$, $A_{22}$, ..., $A_{2(n-2)}$, and $A_{2(n-1)}$), ..., $A_{(n-1)}$ ($A_{(n-1)1}$, $A_{(n-1)2}$, ..., $A_{(n-1)(n-2)}$, and $A_{(n-1)(n-1)}$), and $A_n$ ($A_{n1}$, $A_{n2}$, ..., $A_{n(n-2)}$, and $A_{n(n-1)}$) of candidate elements $A_1$, $A_2$, ..., $A_{(n-1)}$, and $A_n$ and the target plot P ($P_1$, $P_2$, ..., $P_{(n-2)}$, $P_{(n-1)}$) are shown on the distribution information.

Here, when the plot $A_1$ is the origin, the plots $A_2$, ..., $A_{(n-1)}$, and $A_n$ can be respectively represented by vectors $a_2$, ..., $a_{(n-1)}$, $a_n$ (($a_{21}$, $a_{22}$, ..., $a_{2(n-2)}$, and $a_{2(n-1)}$), ..., ($a_{(n-1)1}$, $a_{(n-1)2}$, ..., $a_{(n-1)(n-2)}$, and $a_{(n-1)(n-1)}$), and ($a_{n1}$, $a_{n2}$, ..., $a_{n(n-2)}$, and $a_{n(n-1)}$) = ($A_{21}$-$A_{11}$, $A_{22}$-$A_{12}$, ..., $A_{2(n-2)}$-$A_{1(n-2)}$, and $A_{2(n-1)}$-$A_{1(n-1)}$), ..., ($A_{(n-1)1}$-$A_{11}$, $A_{(n-1)2}$-$A_{12}$, ..., $A_{(n-1)(n-2)}$-$A_{1(n-2)}$, and $A_{(n-1)(n-1)}$-$A_{1(n-1)}$), and ($A_{n1}$-$A_{11}$, $A_{n2}$-$A_{12}$, ..., $A_{n(n-2)}$-$A_{1(n-2)}$, and $A_{n(n-1)}$-$A_{1(n-1)}$)), and the target plot P can be represented by the vector p (($p_1$, $p_2$, ..., $p_{(n-2)}$, and $p_{(n-1)}$) = ($P_1$-$A_{11}$, $P_2$-$A_{12}$, ..., $P_{(n-2)}$-$A_{1(n-2)}$, and $P_{(n-1)}$-$A_{1(n-1)}$)). Note that the plot $A_1$ can be represented by a zero vector $a_1$ (0, 0, ..., 0, and 0). At this time, the vector p can be represented by the following formula using the vectors $a_2$, ..., $a_{(n-1)}$, and $a_n$ and coefficients $\beta_1$, ..., $\beta_{(n-2)}$, and $\beta_{(n-1)}$.

$$p = \beta_1 a_2 + \ldots + \beta_{(n-2)} a_{(n-1)} + \beta_{(n-1)} a_n$$

This formula can be converted into the following formula using a coefficient matrix D.

(Math. 3)

$$\begin{pmatrix} p_1 \\ \vdots \\ p_{(n-1)} \end{pmatrix} = D \begin{pmatrix} \beta_1 \\ \vdots \\ \beta_{(n-1)} \end{pmatrix}$$

$$\begin{pmatrix} \beta_1 \\ \vdots \\ \beta_{(n-1)} \end{pmatrix} = D^{-1} \begin{pmatrix} p_1 \\ \vdots \\ p_{(n-1)} \end{pmatrix}$$

Wherein, $\quad D = \begin{pmatrix} a_{21} & \cdots & a_{2(n-1)} \\ \vdots & \ddots & \vdots \\ a_{n1} & \cdots & a_{n(n-1)} \end{pmatrix}$

In this case, in the case where all the following conditions are satisfied, the composition ratio $A_1:A_2:...:A_{(n-1)}:A_n$ of the candidate elements $A_1, A_2, ..., A_{(n-1)}$, and $A_n$ can be calculated as "$1 - (\beta_1 + \beta_2 + ... + \beta_{(n-2)}$, and $\beta_{(n-1)}) : \beta_1 :...: \beta_{(n-2)}:\beta_{(n-1)}$".

(Math. 4)

$$\sum_{k=1}^{n-1} \beta_k < 1$$

$$\beta_k < 1$$

Further, the index m is calculated from the coefficients $\beta_1, ..., \beta_{(n-2)}$, and $\beta_{(n-1)}$ obtained above on the basis of the following formula.

(Math. 5)

$$m = \sum_{k=1}^{n-1} \left( \beta_k - \frac{1}{n} \right)^2$$

As the index m of the composition becomes smaller, the ratio of each candidate element is closer, making it possible to predict the composition of an alloy that will provide a high reaction rate, which is difficult to predict from the feature amount energy of a single element. Therefore, as a prediction result of the composition of an alloy having a close ratio of each candidate element, a predetermined number of compositions can be listed in ascending order of the index m.

(Step S07: Creation of slab model)

In Step S07, a plurality of slab models corresponding to the plurality of compositions predicted in Step S06 as having a high possibility of providing a high reaction rate is created to calculate the reaction rate and the stability index. From the viewpoint of efficiency, it is favorable to use an existing database to create the slab model. As such a database, it is favorable to use a DFT calculation database such as CatApp, which stores structural data of a slab model that is easy to use for quantum chemical calculations. Note that in addition to the DFT calculation database, a crystal structure database such as Materials Project can be used as a database. Further, in Step S07, a unit cell may be obtained from an existing database for the composition predicted in Step S06, and a slab model may be created on the basis of the acquired unit cell.

Further, for alloys for which no database exists, a slab model can be created independently. The method of creating a slab model is not limited to a specific method, but the following method can be used as an example. In the method of creating a slab model according to an example, first, a first unit cell model is created, which is a model of a unit cell for a crystal structure (a face-centered cubic lattice, a body-centered cubic lattice, a hexagonal close-packed structure, or the like) that is stable when a predetermined element constituting candidate elements contained in each composition is present alone. Next, a second unit cell model is created by substituting some atoms constituting the first unit cell model with atoms of the candidate element other than the predetermined element. The slab model is obtained by creating the second unit cell model with a crystal plane (the (111) plane, the(211) plane, or the like) that is stable when the candidate element constituting the first unit cell model is present alone. Note that the crystal structure and crystal plane that are stable when a metal element is present alone can be obtained from a crystal structure database such as Materials Project.

(Step S08: Calculation of stability index)

In Step S08, the stability index indicating the stability of the structure is calculated for each slab model created in Step S07. The stability of the structure in each slab model is evaluated on the basis of the energy of the slab model. The energy used to evaluate the stability of the structure of the slab model only needs to be an energy that can be calculated from the slab model. Examples of the energy include the total energy of the slab model and the surface energy of the slab model. Specifically, a general physical and chemical calculation method can be used to evaluate the stability of the slab model. Examples of the physical and chemical calculation method that can be used to evaluate the stability of the slab model include the Monte Carlo method.

The stability index only needs to be capable of comparing the stability of the structure between the slab models, and a common index for all slab models is used. Specifically, as the stability index, for example, the energy calculated from the slab model can be used as it is. In this case, it can be seen that the smaller the energy as the stability index, the higher the stability of the structure of the slab model. Further, in addition thereto, other values calculated on the basis of the energy calculated from the slab model can also be used as the stability index.

(Step S09: Calculation of activity index)

In Step S09, the activity index indicating the activity of the catalytic reaction is calculated for each slab model created in Step S07. The activity of the catalytic reaction in each slab model is evaluated on the basis of the reaction rate. Specifically, in Step S09, first, the feature amount energy is calculated for each slab model in the same manner as that in Step S02. Then, for each slab model, the reaction rate corresponding to the feature amount energy is obtained using the distribution information created in Step S04.

The activity index only needs to be capable of comparing the activity between the slab models, and a common index for all slab models is used. Specifically, as the activity index, for example, the reaction rate calculated from the slab model can be used as it is. In this case, it can be seen that the larger the reaction rate as the activity index, the larger the actual reaction rate (activity) of the slab model. Further, in addition thereto, other values calculated on the basis of the reaction rate calculated from the slab model can also be used as the activity index.

(Step S10: Identification of alloy)

In Step S10, an alloy that is expected to have both high stability of the structure and high activity of the catalytic reaction is identified on the basis of the stability index of each slab model calculated in Step S08 and the activity index of each slab model calculated in Step S09. In Step S10, various method can be used to identify an alloy from the stability index and the activity index of each slab model.

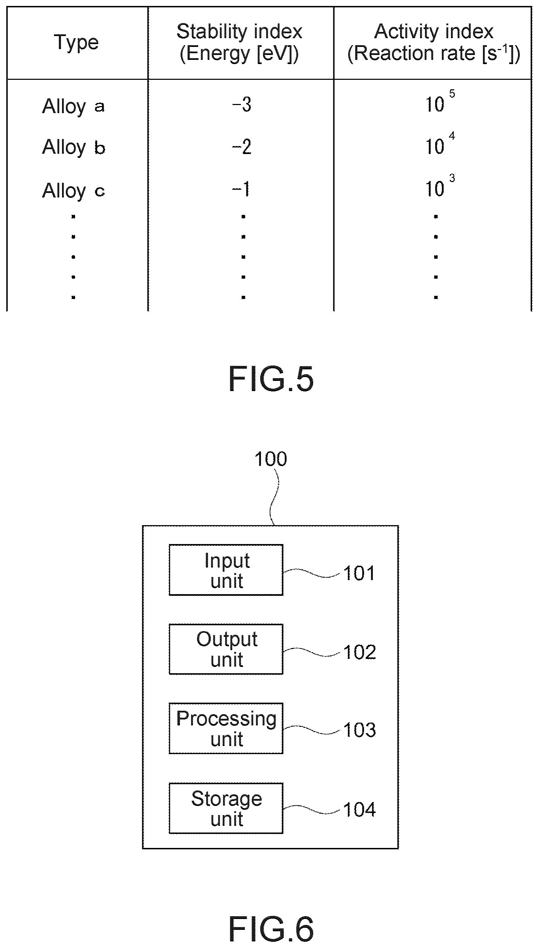

As an example, a list of alloys as shown in Fig. 5 can be used to identify an alloy. In the list of alloys shown in Fig. 5, pieces of data showing the stability index (energy) and the activity index (reaction rate) for each type of alloy identified by the slab model are listed. For example, in the list of alloys, a plurality of alloys can be shown in descending order of the activity index. In this case, for example, a threshold value for the stability index is determined in advance, alloys whose stability index is larger than the threshold value are excluded from the plurality of alloys, and then, a predetermined number of alloys can be selected in order from the top of the list of alloys. The threshold value of the stability index is favorably set to a sufficiently small value so that high stability of the structure can be achieved. Further, in the list of alloys, the plurality of alloys can be arranged from the top in descending order of the stability index. In this case, for example, a threshold value for the activity index is determined in advance, alloys whose activity index is smaller than the threshold value are excluded from the plurality of alloys, and then, a predetermined number of alloys can be selected in order from the top of the list of alloys. The threshold value of the activity index is favorably set to a sufficiently large value so that the catalytic reaction can proceed smoothly.

(Other embodiments)

In the screening method according to this embodiment, various modifications can be made to the above configuration within the scope in which the above effects can be appropriately achieved.

For example, in Step S09, the activity index may be calculated for only the slab model whose stability index was good in Step S08. This reduces the calculation load in Step S09 and narrows down the candidates for slab models when identifying the alloy in Step S10. Further, the order of Step S08 and Step S09 may be reversed, i.e., the stability index of each slab model may be calculated in Step S08 after the activity index of each slab model is calculated in Step S09. In this case, in Step S08, the stability index may be calculated for only the slab model whose activity index was good in Step S09.

[Additional configuration of screening method]

(Overview)

The screening method according to this embodiment is not limited to the configuration described above and may have an additional configuration for further improving the screening accuracy of alloys. An example of an effective additional configuration for the screening method according to this embodiment will be described below.

(Searching for composition of alloy using selectivity as index)

Although the activity index is used as an output in the above embodiment, distribution information (heatmap) of selectivity can be described in a manner similar to that for the above activity index in the case where the same feature amount energy is obtained for catalytic reactions with two or more reaction paths. In this case, in addition to the main reaction activity, what percent is the selectivity of the main reaction product can be added to the output. A selectivity S can be defined as follows when, for example, the products are i and j and their reaction rates are $r_i$ and $r_j$.

$$S = r_i / (r_i + r_j)$$

(Further searching for composition of alloy)

In the above embodiment, distribution information created for a crystal structure formed of a single metal is used to identify an alloy formed of a plurality of metals. That is, when identifying the alloy, the interactions between metals that occur in the actual alloy are not taken into consideration. For this reason, there is a possibility that the composition of the alloy identified in the above embodiment is shifted slightly from the composition that will actually provide the highest reaction rate. Therefore, there is a possibility that by further searching for a composition close to the composition of the alloy identified in the above embodiment, an alloy with even higher activity of the catalytic reaction can be found. Such searching for a composition can be carried out by, for example, creating the slab model and evaluating the stability index for a plurality of alloys whose compositions are shifted from that of the alloy identified in the above embodiment.

As an example, a plurality of alloys in which the composition ratio of each candidate metal is increased or decreased by one with respect to the composition of the alloy identified in the above embodiment can be searched for. For example, in the case where the composition of the alloy identified in the above embodiment is a ternary system including the candidate elements A, B, and C in a composition ratio of A:B:C, alloys with 6 composition ratios of A+1:B-1:C, A+1:B:C-1, A:B+1:C-1, A:B-1:C+1, A-1:B+1:C, and A-1:B:C+1 are to be searched for. For the plurality of alloys to be searched for, a slab model is created in the same manner as that in Step S07, a reaction rate is obtained in the same manner as that in Step S09, and an alloy with the highest reaction rate is identified. Further, by repeating the same search for the alloy identified in this way, it is conceivable that the obtained composition is closer to the composition of the alloy that will provide the highest reaction rate. Such a search for a composition can be repeated, for example, until the rate of increase in the reaction rate relative to the previously identified alloy becomes a predetermined numerical value or less, i.e., it is determined that the reaction rate has converged. Further, the search for a composition may be repeated a predetermined number of times.

(Evaluation using nanoparticle model)

Although the stability of the structure of an alloy is evaluated using a slab model in the above embodiment, the stability of the structure of an alloy cannot be accurately evaluated in some cases using a slab model that focuses only on a single crystal plane. In this regard, using a nanoparticle model that expresses the three-dimensional structure of alloy nanoparticles allows the stability of the structure of the alloy to be evaluated more accurately. For this reason, the screening method according to this embodiment can incorporate the evaluation using a nanoparticle model of an alloy to evaluate the stability of the alloy more reliably. A known modeling method can be used to create the nanoparticle model of an alloy.

Examples of the method of modeling catalyst nanoparticles according to the composition of an alloy slab model include a genetic algorithm method.

The stability of the structure in each nanoparticle model is evaluated on the basis of the energy of the nanoparticle model. The energy to be used to evaluate the stability of the structure in the nanoparticle model only needs to be an energy that can be calculated from the nanoparticle model. Examples thereof include the energy of the nanoparticle model. A physical and chemical calculation method similar to that for the evaluation of the stability of the slab model in Step S08 can be used to calculate the energy of the nanoparticle model. The result obtained from the nanoparticle model can be used as a stability index indicating the stability of the structure of the alloy. This stability index can be used instead of the stability index calculated in Step S08 and can be used as a second stability index together with the stability index calculated in Step S08.

As an example, for each slab model created in Step S07, a nanoparticle model in which a plurality of candidate elements constituting the slab model is common is created. Then, nanoparticle information is created in which the energy, the composition ratio, and coordinates of all atoms are associated with each other for each nanoparticle model. By comparing the peripheral information of a specific atom obtained from the coordinates of all atoms in the nanoparticles with the peripheral information of a specific atom obtained from the coordinates of all atoms constituting the slab model, the similarity can be calculated. Examples of the peripheral information of a specific atom include the coordination number and the radial distribution function, and examples of the similarity include the difference in the coordination number and the integral value of the difference in the radial distribution function. The nanoparticle model with the highest similarity for each slab model is identified, and the energy of nanoparticle model corresponding to each slab model is adopted. Then, it can be seen that the alloy with a smaller energy obtained from the nanoparticle information has higher stability of the structure.

[Information processing apparatus]

The screening method according to the above embodiment can be realized by, for example, using an information processing apparatus 100 shown in Fig. 6. The information processing apparatus 100 includes an input unit 101, an output unit 102, a processing unit 103, and a storage unit 104.

The information processing apparatus 100 according to this embodiment may include various computers, and may be configured with a single computer or a combination of two or more computers.

The functions realized by the components described in the present specification may be implemented in a circuitry or processing circuitry including a general-purpose processor, an application-specific processor, an integrated circuit, ASICs (Application Specific Integrated Circuits), CPU (a Central Processing Unit), an existing circuit, and/or combinations thereof, which are programmed to realize the described functions. The processor includes a transistor and other circuits and is regarded as a circuitry or processing circuitry. The processor may be a programmed processor that executes a program stored in a memory.

In the present specification, the circuitry, unit, and means are hardware programmed to realize the described functions or hardware that executes the described functions. The hardware may be any hardware disclosed in the present specification or any hardware known to be programmed to realize the described functions or execute the described functions.

In the case where the hardware is a processor that is regarded as a circuitry type, the circuitry, means, or unit is a combination of hardware and software to be used for configuring the hardware and/or processor.

The input unit 101 is configured as a user interface, and is used to input, for example, a target catalytic reaction, a plurality of elementary reactions, a plurality of candidate elements, and a crystal plane in Step S01. The output unit 102 is configured to be capable of outputting information to a display device such as various displays, and can be configured to be capable of displaying, for example, at least one of the distribution information created in Step S04 and the alloy index used in Step S10 (a stability index and an activity index). The processing unit 103 is configured to be capable of executing all or part of the processes included in the screening method according to the above embodiment in accordance with a program. The storage unit 104 is configured to be capable of storing various types of data, and stores, for example, a program for causing the processing unit 103 to execute each process and various types of data necessary for each process by the processing unit 103. Further, the information processing apparatus 100 may have a configuration other than the above, and may include, for example, a communication unit for acquiring data from a database on the cloud through communication.

[Other embodiments]

Although an embodiment of the present invention has been described above, the present invention is not limited to only the above-mentioned embodiment, and it goes without saying that various modifications can be made without departing from the essence of the present invention.

Reference Signs List

100 information processing apparatus
101 input unit
102 output unit
103 processing unit
104 storage unit

**Claims**

1. A screening method for an alloy that causes a target catalytic reaction, comprising:

creating, for each of a plurality of candidate elements that is a candidate for a metal element constituting the alloy, basic information including respective states of each reaction substrate in a non-adsorbed state and an energy and a vibration frequency of each reaction substrate in an adsorbed state and each atom constituting the reaction substrate in a plurality of elementary reactions constituting the catalytic reaction, for a specific crystal plane formed by each of the candidate elements;
obtaining, for each of the plurality of candidate elements, a plurality of activation energies corresponding to the plurality of elementary reactions, a plurality of differential energies obtained as relative values of energies in the respective states, and a reaction rate of the catalytic reaction from the basic information;
evaluating, for each activation energy constituting the plurality of activation energies and each differential energy constituting the plurality of differential energies, linearity from a relationship between the plurality of candidate elements and selecting, from the plurality of differential energies, a feature amount energy from which high linearity is obtained for all of the plurality of activation energies;
creating distribution information indicating distribution of the reaction rate with respect to the feature amount energy using the feature amount energy and the reaction rate in the plurality of candidate elements;
predicting, by using the distribution information, a plurality of compositions that contains at least two candidate elements of the plurality of candidate elements and increases the reaction rate when forming the alloy;
creating a plurality of slab models corresponding to the plurality of compositions;
calculating a stability index on a basis of an energy calculated for each of the plurality of slab models; and
calculating an activity index on a basis of the reaction rate obtained from the distribution information using the feature amount energy calculated for each of the plurality of slab models.

2. The screening method according to claim 1, wherein
the feature amount energy includes an adsorption energy.

3. The screening method according to claim 1 or 2, wherein

the feature amount energy includes two feature amount energies different from each other and
the distribution information is created as a two-dimensional heatmap.

4. The screening method according to any one of claims 1 to 3, further comprising
setting a target plot on the distribution information to predict the plurality of compositions, selecting plots of the at least two candidate elements, and predicting, by using coordinate information of the target plot and the plots of the candidate elements, the at least two candidate elements from which the reaction rate close to that of the target plot is obtained and a composition ratio thereof.

5. The screening method according to any one of claims 1 to 4, further comprising

acquiring, from a database, a unit cell containing the at least two candidate elements and creating the plurality of slab models on a basis of the acquired unit cell, or
creating a first unit cell model for a crystal structure that is stable when a predetermined element constituting the at least two candidate elements is present alone, creating a second unit cell model obtained by substituting some atoms constituting the first unit cell model with atoms of the at least two candidate elements other than the predetermined element, and creating the plurality of slab models on a basis of the second unit cell model.

6. The screening method according to any one of claims 1 to 5, further comprising
creating a nanoparticle model corresponding to the at least two candidate elements, calculating a composition ratio and an energy corresponding to peripheral information of a specific atom for the nanoparticle model, and obtaining an energy of the nanoparticle model corresponding to each of the plurality of slab models on a basis of a composition ratio and peripheral information of a specific atom of each of the plurality of slab models to calculate the stability index.

7. An information processing apparatus that performs screening for an alloy that causes a target catalytic reaction, comprising:
a processing unit that executes

creating, for each of a plurality of candidate elements that is a candidate for a metal element constituting the alloy, basic information including respective states of each reaction substrate in a non-adsorbed state and an energy and a vibration frequency of each reaction substrate in an adsorbed state and each atom constituting the reaction substrate in a plurality of elementary reactions constituting the catalytic reaction, for a specific crystal plane formed by each of the candidate elements,
obtaining, for each of the plurality of candidate elements, a plurality of activation energies corresponding to the plurality of elementary reactions, a plurality of differential energies obtained as relative values of energies in the respective states, and a reaction rate of the catalytic reaction from the basic information,
evaluating, for each activation energy constituting the plurality of activation energies and each differential energy constituting the plurality of differential energies, linearity from a relationship between the plurality of candidate elements and selecting, from the plurality of differential energies, a feature amount energy from which high linearity is obtained for all of the plurality of activation energies,
creating distribution information indicating distribution of the reaction rate with respect to the feature amount energy using the feature amount energy and the reaction rate in the plurality of candidate elements,
predicting, by using the distribution information, a plurality of compositions that contains at least two candidate elements of the plurality of candidate elements and increases the reaction rate when forming the alloy,
creating a plurality of slab models corresponding to the plurality of compositions,
calculating a stability index on a basis of an energy calculated for each of the plurality of slab models, and calculating an activity index on a basis of the reaction rate obtained from the distribution information using the feature amount energy calculated for each of the plurality of slab models.

8. The information processing apparatus according to claim 7, wherein
the feature amount energy selected by the processing unit includes an adsorption energy.

9. The information processing apparatus according to claim 7 or 8, wherein
the feature amount energy includes two feature amount energies different from each other and the distribution information is created as a two-dimensional heatmap in the processing unit.

10. The information processing apparatus according to any one of claims 7 to 9, wherein
the processing unit sets a target plot on the distribution information to predict the plurality of compositions, selects plots of the at least two candidate elements, and predicts, by using coordinate information of the target plot and the plots of the candidate elements, the at least two candidate elements from which the reaction rate close to that of the target plot is obtained and a composition ratio thereof.

11. The information processing apparatus according to any one of claims 7 to 10, wherein
the processing unit

acquires, from a database, a unit cell including the at least two candidate elements and creates the plurality of slab models on a basis of the acquired unit cell, or
creates a first unit cell model for a crystal structure that is stable when a predetermined element constituting the at least two candidate elements is present alone, creates a second unit cell model obtained by substituting some atoms constituting the first unit cell model with atoms of the at least two candidate elements other than the predetermined element, and creates the plurality of slab models on a basis of the second unit cell model.

12. The information processing apparatus according to any one of claims 7 to 11, wherein
the processing unit creates a nanoparticle model corresponding to the at least two candidate elements, calculates a composition ratio and an energy corresponding to peripheral information of a specific atom for the nanoparticle model, and obtains an energy of the nanoparticle model corresponding to each of the plurality of slab models on a basis of a composition ratio and peripheral information of a specific atom of each of the plurality of slab models to calculate the stability index.

13. The information processing apparatus according to any one of claims 7 to 12, wherein

the feature amount energy includes two feature amount energies different from each other in the processing unit, the information processing apparatus further comprising an output unit that outputs the distribution information as a two-dimensional heatmap.

14. The information processing apparatus according to any one of claims 7 to 13, further comprising an output unit capable of displaying at least one of the distribution information created by the processing unit or the stability index and the activity index calculated by the processing unit.

15. A program for performing screening for an alloy that causes a target catalytic reaction and causing an information processing apparatus to execute:

creating, for each of a plurality of candidate elements that is a candidate for a metal element constituting the alloy, basic information including respective states of each reaction substrate in a non-adsorbed state and an energy and a vibration frequency of each reaction substrate in an adsorbed state and each atom constituting the reaction substrate in a plurality of elementary reactions constituting the catalytic reaction, for a specific crystal plane formed by each of the candidate elements;
obtaining, for each of the plurality of candidate elements, a plurality of activation energies corresponding to the plurality of elementary reactions, a plurality of differential energies obtained as relative values of energies in the respective states, and a reaction rate of the catalytic reaction from the basic information;
evaluating, for each activation energy constituting the plurality of activation energies and each differential energy constituting the plurality of differential energies, linearity from a relationship between the plurality of candidate elements and selecting, from the plurality of differential energies, a feature amount energy from which high linearity is obtained for all of the plurality of activation energies;
creating distribution information indicating distribution of the reaction rate with respect to the feature amount energy using the feature amount energy and the reaction rate in the plurality of candidate elements;
predicting, by using the distribution information, a plurality of compositions that contains at least two candidate elements of the plurality of candidate elements and increases the reaction rate when forming the alloy;
creating a plurality of slab models corresponding to the plurality of compositions;
calculating a stability index on a basis of an energy calculated for each of the plurality of slab models; and
calculating an activity index on a basis of the reaction rate obtained from the distribution information using the feature amount energy calculated for each of the plurality of slab models.

FIG.1

## FIG.2

## FIG.3

## FIG.4

| Type | Stability index (Energy [eV]) | Activity index (Reaction rate [$s^{-1}$]) |
|---|---|---|
| Alloy a | −3 | $10^5$ |
| Alloy b | −2 | $10^4$ |
| Alloy c | −1 | $10^3$ |
| . | . | . |
| . | . | . |
| . | . | . |
| . | . | . |
| . | . | . |

## FIG.5

100

| |
|---|
| Input unit —101 |
| Output unit —102 |
| Processing unit —103 |
| Storage unit —104 |

## FIG.6

## INTERNATIONAL SEARCH REPORT

| International application No. |
| --- |
| **PCT/JP2022/048515** |

### A. CLASSIFICATION OF SUBJECT MATTER

*B01J 37/00*(2006.01)i
FI: B01J37/00 Z

According to International Patent Classification (IPC) or to both national classification and IPC

### B. FIELDS SEARCHED

Minimum documentation searched (classification system followed by classification symbols)

B01J21/00-38/74;C22C1/00-1/12;B22F1/00-12/90

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JST7580/ JSTChina (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| A | WO 2022/138270 A1 (TANAKA KIKINZOKU KOGYO KK) 30 June 2022 (2022-06-30) claim 15, paragraphs [0035]-[0070] | 1-15 |
| A | YU, Ya-Xin et al. High-Throughput Screening of Alloy Catalysts for Dry Methane Reforming. ACS Catalysis. 2021, vol. 11, pp. 8881-8894, DOI: 10.1021/acscatal.0c04911 entire text, all drawings | 1-15 |
| A | US 2020/0168300 A1 (CALIFORNIA INSTITUTE OF TECHNOLOGY) 28 May 2020 (2020-05-28) claims, examples | 1-15 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **07 March 2023** | **20 March 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/048515**

| Patent document cited in search report | | | Publication date (day/month/year) | Patent family member(s) | | | Publication date (day/month/year) |
|---|---|---|---|---|---|---|---|
| WO | 2022/138270 | A1 | 30 June 2022 | JP | 2022-98687 | A | |
| US | 2020/0168300 | A1 | 28 May 2020 | WO | 2020/113136 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020006312 A